# EUROPEAN PATENT APPLICATION

(11) **EP 4 748 321 A1**
(43) Date of publication of application: **27.05.2026**
(21) Application number: 25216865.3
(22) Date of filing: 19.11.2025
(51) Int. Cl.: A61B 17/04

(54) **SOFT SUTURE ANCHOR ASSEMBLEY WITH LOCKING ELEMENT**

(30) Priority: 21.11.2024 US 202463723237 P; 20.01.2025 US 202563747259 P; 17.02.2025 US 202563759401 P; 08.04.2025 US 202563785034 P; 14.05.2025 US 202563805396 P
(71) Applicant: Smith & Nephew Inc., Memphis, TN 38116 (US); Smith & Nephew Asia Pacific Pte. Limited, Singapore 138565 (SG)
(72) Inventor: Housman, Mark Edwin, Memphis, TN 38116 (US); Hall, Benjamin, Memphis, TN 38116 (US); Mirabile, Belin, Memphis, TN 38116 (US)
(74) Representative: Appleyard Lees IP LLP

(57) **Abstract**

A soft suture anchor assembly (100) includes a soft anchor (300) defining a tubular body (302). A locking member (304) has a locking member body (306) coupled directly to and extending distally from the distal end (302b) of the soft anchor (300). The locking member body (306) defines at least one passage (308) therethrough. At least one transfer member (400) extends along the tubular body (302) and through the at least one passage (308) of the locking member (304). The at least one transfer member (400) is configured to draw at least one repair suture through the at least one passage (308) in a first direction. The at least one passage (308) is configured to allow the at least one repair suture to slide freely in the first direction through the at least one passage (308) and to prevent the at least one repair suture from sliding freely in a second direction opposite the first direction through the at least one passage (308).

## Description

### CROSS-REFERENCED TO RELATED APPLICATIONS

This application claims priority to and benefit of U.S. Provisional Application No. 63/723,237, filed November 21, 2024, entitled ALL-SUTURE ANCHOR WITH REPAIR SUTURE LOCKING ELEMENT; U.S. Provisional Application No. 63/747,259, filed January 20, 2025, entitled ALL-SUTURE ANCHOR WITH LOCKING ELEMENT; U.S. Provisional Application No. 63/759,401, filed February 17, 2025, entitled ALL-SUTURE ANCHOR WITH LOCKING ELEMENT; U.S. Provisional Application No. 63/785,034, filed April 8, 2025, entitled ANCHOR WITH KNOTLESS LOCKING ELEMENT; and U.S. Provisional Application No. 63/805,396, filed May 14, 2025, entitled ANCHOR WITH KNOTLESS LOCKING ELEMENT, the entire contents of which are incorporated herein by reference for all purposes.

### FIELD

The present disclosure relates generally to soft-bodied suture anchors and, more particularly, to soft-bodied suture anchors containing a suture locking member.

### BACKGROUND

Surgeons often use soft suture anchors during tissue repair because they allow for placement of anchors in areas of the body where only a small anchor can be used, and because use of such anchors can avoid placing plastic or metal within the body. Soft suture anchors typically contain a tubular or sheath-like body made of a soft, flexible material, and a suture interwoven throughout the body or otherwise coupled to the body. When the anchor is in an undeployed, insertable state, it is typically elongate such that a length of the body is greater than its width. Tension applied to the suture causes the body to expand radially and shorten axially, forming an interference fit within the surrounding tissue, such as a bone hole. Thus, such anchors can be used to fix soft tissue in a repair without the need to tie knots in the suture.

### SUMMARY

Unlike traditional hard-bodied anchors, current knotless soft suture anchors may only capture and secure repair sutures originating from another knotless anchor. Managing the sutures in such a suture construct can be challenging and deviates from the workflow typically used with traditional hard anchors. The present disclosure advantageously allows the user to mimic the conventional workflow more closely, simplifying suture management and reducing associated difficulties when creating more complex knotless anchor constructs.

This disclosure describes a soft suture anchor assembly that includes a locking element configured to receive an external repair suture and lock it into place. The locking element may be a separately formed element and may be a one-way locking member. The locking element may be at a distal end of the soft anchor and may be fixedly attached thereto. The locking element may have portion that is rigid. The repair suture may be fabricated to preferably lock with the locking element.

Embodiments of the soft suture anchor assembly of this disclosure may include one or more of the following, in any suitable combination.

In embodiments, a soft suture anchor assembly for tissue repair of this disclosure includes a soft anchor defining a tubular body. A locking member having a locking member body is coupled directly to and extends distally from a distal end of the tubular body. The locking member body defines at least one passage therethrough. At least one transfer member extends along the tubular body and through the at least one passage of the locking member body. The at least one transfer member is configured to draw at least one repair suture through the at least one passage in a first direction. The at least one passage is configured to allow the at least one repair suture to slide freely in the first direction through the at least one passage and to prevent the at least one repair suture from sliding freely in a second direction opposite the first direction through the at least one passage.

In further embodiments, the tubular body is configured to deploy from an elongated to an expanded state within tissue. In embodiments, the at least one transfer member is configured to draw the at least one repair suture through the at least one passage in the first direction after deploying the tubular body to the expanded state. In embodiments, the at least one transfer member is configured to draw the at least one repair suture through the at least one passage in the first direction after insertion of the locking member into tissue. In embodiments, the at least one transfer member extends along and is interwoven through the tubular body and is configured to draw the at least one repair suture along and between braids of braided walls of the tubular body. In embodiments, an end of the at least one transfer member is configured to couple to the at least one repair suture by a loop. In embodiments, the repair suture originates from another knotless anchor. In embodiments, the at least one passage includes at least one suture locking element configured to engage the at least one repair suture to prevent the at least one repair suture from sliding freely in the second direction. In embodiments, the at least one suture locking element is at least one tab, flexure, wire, or tooth on an inner surface of the at least one passage. In embodiments, the at least one suture locking element is a ball configured to wedge the repair suture between the ball and an inner surface of the passage. In embodiments, the at least one passage is at least one through passage in communication with at least one suture capture passage. In embodiments, the locking member has a pointed tip. In embodiments, the repair suture has a middle region extending between two end regions. In embodiments, a number of carriers of the two end regions is selected to be fewer than a number of carriers of the middle region, and the carriers of the two end regions are braided at higher picks per inch (PPI) than the carriers of the middle region.

Embodiments of a method of tissue repair of this disclosure include inserting a locking member of a soft suture anchor assembly into tissue. The soft suture anchor assembly includes a soft anchor defining a tubular body. The locking member includes a locking member body coupled directly to and extending distally from a distal end of the tubular body. The locking member body defines at least one passage therethrough. The at least one passage is configured to allow at least one repair suture to slide freely in a first direction through the at least one passage and to prevent the at least one repair suture from sliding freely in a second direction opposite the first direction through the at least one passage. At least one transfer member extends along the tubular body and through the at least one passage of the locking member. The method also includes, using the transfer member, drawing at least one repair suture through the at least one passage of the locking member in the first direction. The method also includes tensioning the repair suture by drawing the at least one repair suture through the at least one passage in the second direction, whereby a suture locking element of the locking member prevents the at least one repair suture from sliding freely through the passage in the second direction.

In further embodiments, the method includes drawing the at least one repair suture along and between braids of braided walls of the tubular body with the at least one transfer member. In embodiments, the method includes, before drawing the at least one repair suture through the at least one passage of the locking member in the first direction, deploying the soft anchor from an elongated state to an expanded state within the tissue. In embodiments, the at least one suture locking element is at least one tab, flexure, wire, or tooth on an inner surface of the at least one passage. In embodiments, the at least one suture locking element is a ball configured to wedge the repair suture between the ball and an inner surface of the passage. In embodiments, drawing the at least one repair suture through the at least one passage of the locking member in the first direction includes drawing the at least one repair suture originating from another knotless anchor.

A reading of the following detailed description and a review of the associated drawings will make apparent these and other features and advantages. Both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIGS. 1A and 1B illustrate a soft suture anchor assembly for tissue repair, according to an embodiment of the disclosure;
FIGS. 1C-1F illustrate various configurations of the locking member of the soft suture anchor assembly, according to embodiments of the disclosure;
FIGS. 1G and 1H are schematic illustrations of the soft anchor, the locking member, and the transfer member of the soft suture anchor assembly, according to embodiments of the disclosure;
FIGS. 2A and 2B illustrate an alternative locking member for use with the soft suture anchor assembly, according to an embodiment of the disclosure;
FIGS. 3A and 3B illustrate another alternative locking member for use with the soft suture anchor assembly, according to an embodiment of the disclosure;
FIGS. 3C-3G illustrate the action of the flexures of the locking member of FIGS. 3A and 3B, according to an embodiment of the disclosure;
FIGS. 4A and 4B illustrate another alternative locking member for use with the soft suture anchor assembly, according to an embodiment of the disclosure;
FIGS. 5A and 5B illustrate another alternative locking member 804 for use with the soft suture anchor assembly, according to an embodiment of the disclosure;
FIG. 5C illustrates an alternative configuration of the locking member of FIGS. 5A and 5B, according to an embodiment of the disclosure;
FIG. 5D is a schematic illustration of the soft anchor, the locking member, and two transfers members according to an embodiment of the disclosure;
FIG. 6A is a schematic illustration of a repair suture for use with the soft suture anchor assembly, according to an embodiment of the disclosure;
FIGS. 6B and 6C are detailed illustrations of the end regions (FIG. 6B) and the middle region (FIG. 6C) of the repair suture of FIG. 6A, according to an embodiment of the disclosure; and
FIG. 7 is a flowchart of a method of tissue repair using the soft suture anchor assembly 100, according to an embodiment of the disclosure.

### DETAILED DESCRIPTION

In the description that follows, like components have been given the same reference numerals, regardless of whether they are shown in different embodiments. To illustrate embodiment(s) in a clear and concise manner, the drawings may not necessarily be to scale, and certain features may be shown in somewhat schematic form. Features that are described and/or illustrated with respect to one embodiment may be used in the same way or in a similar way in one or more other embodiments and/or in combination with or instead of the features of the other embodiments.

As used in the specification and claims, for the purposes of describing and defining the invention, the term "substantially" is used to represent the inherent degree of uncertainty that may be attributed to any quantitative comparison, value, measurement, or other representation. The term "substantially" is also used herein to represent the degree by which a quantitative representation may vary from a stated reference without resulting in a change in the basic function of the subject matter at issue.

Unless specifically stated or obvious from context, the term "about" is understood as within a range of normal tolerance in the art. Unless otherwise clear from context, all numerical values provided herein are modified by the term "about." Provided ranges are understood to be shorthand for all the values within the range. "Comprise," "include," and/or plural forms of each are open ended and include the listed parts and can include additional parts that are not listed. "And/or" is open-ended and includes one or more of the listed parts and combinations of the listed parts. Use of the relative terms "top," "bottom," "above," "below" and the like helps only in the clear description of the disclosure and does not limit the structure, positioning and/or operation of the disclosure in any manner.

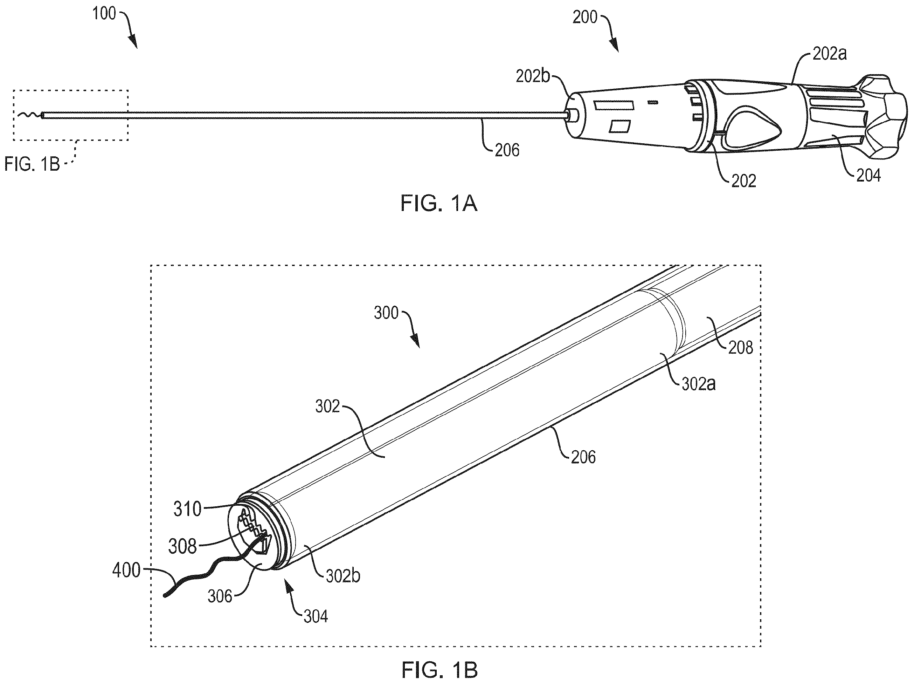

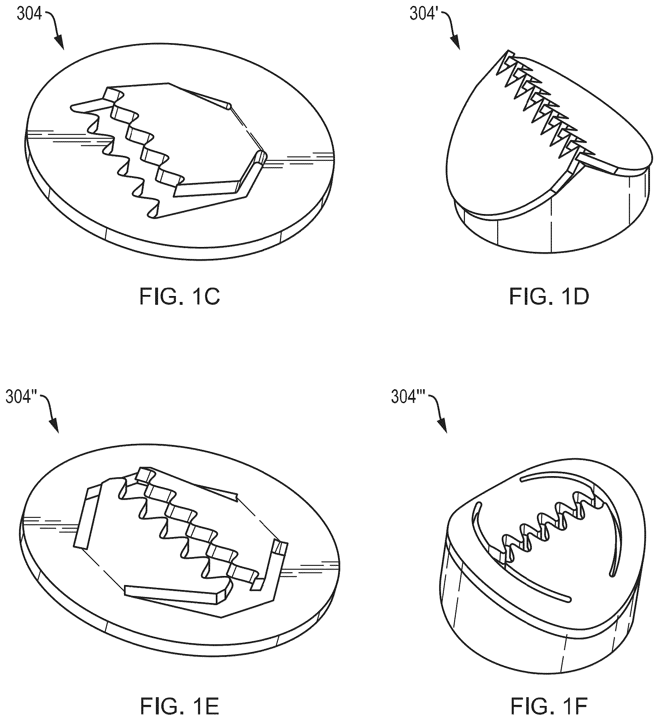

FIGS. 1A and 1B illustrate a soft suture anchor assembly 100 for tissue repair, according to an embodiment of the disclosure. As shown in FIGS. 1A and 1B, the soft suture anchor assembly 100 may generally comprise an insertion instrument 200 that has a handle 202 and a tensioning actuator 204 at a proximal end 202a of the handle 202. A hollow tube 206 may extend from a distal end 202b of the handle 202. A soft anchor 300 may include a tubular body 302 positioned within the hollow tube 206. The tubular body 302 may be made of a soft, flexible material. For example, the tubular body 302 may comprise one of suture, tape, braid, or mesh. Non-limiting examples of soft anchors 300 of this disclosure are described in U.S. Patent No. 9,962,149 to ArthroCare Corporation (Austin, TX), incorporated herein by reference. One or flexible members (not shown) may be operatively coupled to the tubular body 302 of the soft anchor 300. For example, the flexible members may be woven or stitched through the tubular body 302. The flexible members may extend proximally through the hollow tube 206 and may operatively couple to the tensioning actuator 204 such that actuation of the tensioning actuator 204 applies tension on the flexible members. Tensioning the flexible members may deploy the tubular body 302 from a first, elongated state (as shown) to a second expanded state when the tubular body 302 is pushed out of the tube 206 by a pusher member 208 and compressed against the distal end of the tube 206. This expansion of the tubular body 302 fixes the soft anchor 300 within the surrounding tissue.

Still referring to FIGS. 1A and 1B, the tubular body 302 may have a proximal end 302a and a distal end 302b. A locking member 304 may comprise a flat, cylindrical, or other suitably shaped body 306 configured to directly couple to the distal end 302b of the tubular body 302 and defining a passage 308 therethrough. Embodiments of the locking member 304 could be a separate component from the tubular body 302 or could be fused or mechanically integrated into the tubular body 302. Embodiments of the locking member 304 could be external to the tubular body 302 or could be positioned internally within a lumen defined by the tubular body 302. The passage 308 may be include at least one suture locking element configured to allow at least one repair suture to slide freely through the passage 308 in a first (e.g., distal) direction and to engage the at least one repair suture to prevent it from sliding in a second (e.g., proximal) direction. For example, as shown in FIG. 1B, the locking member body 306 may be made of a flexible material (such as flexible sheet metal) and the passage 308 may include bends, teeth or tabs 310 on an inner surface of the passage 308. A transfer member 400 (e.g., a suture or wire) may extend through the passage 308 of the locking member 304 and along the tubular body 302. The transfer member 400 may be configured to draw the repair suture through the passage 308 in the first direction. As the repair suture is pulled distally through the passage 308, the flexible material of the locking member 304 may deflect outwardly to allow passage of the repair suture, but snap back into its original configuration once the pulling force on the repair suture is released. Once the flexible material snaps back to its original configuration, the tabs 310 may grip or trap the repair suture, preventing it from sliding proximally through the tube 206.

FIGS. 1C-1F illustrate various configurations of the locking member 304, 304', 304", 304‴ of the soft suture anchor assembly 100, according to embodiments of the disclosure. In embodiments, the locking members 304, 304', 304" may be made from two or three separate components that are pieced together, such as by laser welded joints. The disclosure also contemplates that the locking member 304‴ could have a single-piece design that includes a single or double flexure.

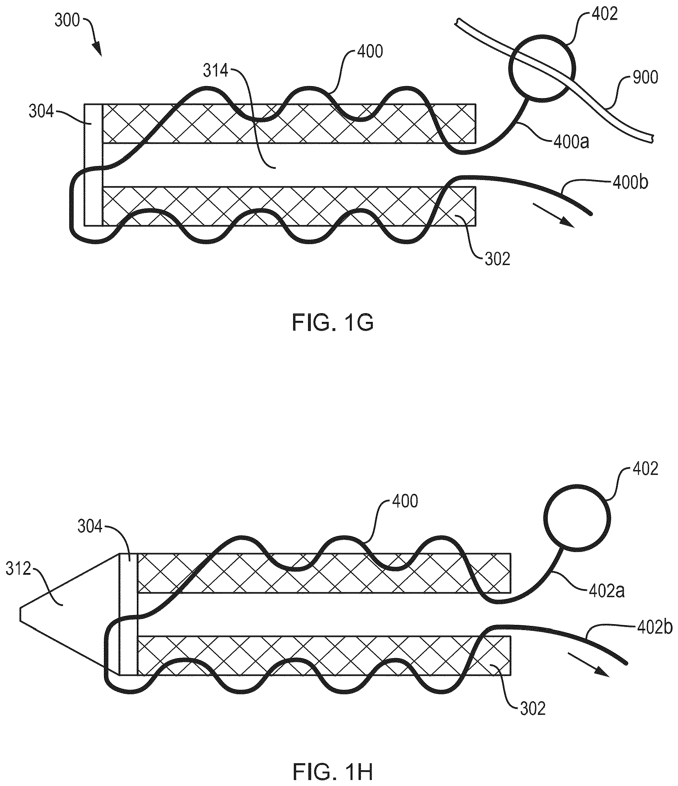

FIGS. 1G and 1H are schematic illustrations of the soft anchor 300, the locking member 304, and the transfer member 400 of the soft suture anchor assembly 100, according to embodiments of the disclosure. As shown in FIG. 1G, the transfer member 400 may be coupled to the tubular body 302 defining a lumen 314 therethrough such that the transfer member 400 extends along the tubular body 302 and through the locking member 304. For example, where the tubular body 302 is made of a braided material, the transfer member 400 may be interwoven between braids of the braided walls of the tubular body 302. The transfer member 400 may extend along a first side of the tubular body 302 in a first direction and extend through the passage 308 of the locking member 304. The transfer member 400 may then extend along a second side of the tubular body 302 in a second direction opposite the first direction. A first end 400a of the transfer member 400 may comprise a loop 402 or other coupling device extending proximally from the tubular body 302 for coupling to a repair suture 900. The repair suture 900 may originate from another knotless anchor, whether soft- or hard-bodied. A user may first insert the locking member 304 into tissue and then deploy the tubular body 302 to its expanded state to lock the soft anchor 300 into the surrounding tissue. The user may then couple the repair suture 900 to the loop 402 and pull a second end 400b of the transfer member 400 such that the repair suture 900 is pulled distally through the passage 308. Once the user releases the pulling force on the transfer member 400, the repair suture 900 may be locked within the passage 308. The repair suture 900 may then be used in the rest of the tissue repair. As shown in FIG. 1H, a pointed tip 312 on the locking member 304 may allow for a "self-punching" version of the locking member 304 that can penetrate tissue without the need for a pre-drilled hole. Such a design may be advantageous when used in a rotator cuff repair where visibility is limited, and bone quality is relatively soft.

The disclosure contemplates that the geometry and material choices for the locking member 304 could be altered to provide various locking strengths on the repair suture 900. For example, tooth geometry, gap spacing, and tooth length and width may affect locking strength. These variables may be selected to best fit and lock onto the desired suture quantity and size. Additionally, adjusting a stiffness of the material of the locking member may adjust the difficulty of passing and locking the repair suture 900. Advantageously, the locking member 304 may maintain performance independent of bone quality. Furthermore, the locking member 304 may allow for final tensioning of the repair suture 900 at any point during the procedure up until the suture is trimmed, which is not possible with many current suture anchors.

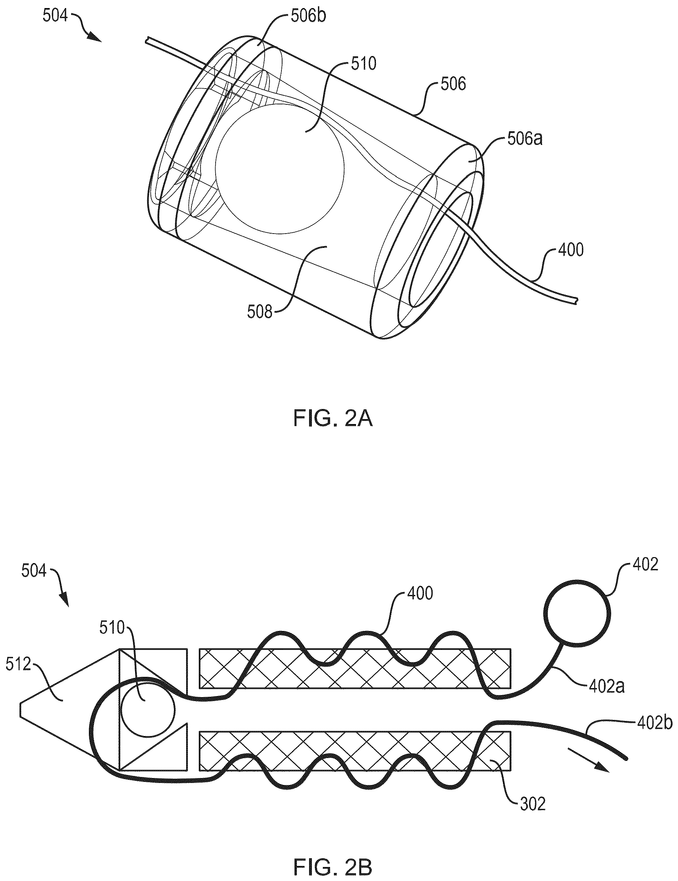

FIGS. 2A and 2B illustrate an alternative locking member 504 for use with the soft suture anchor assembly 100, according to an embodiment of the disclosure. As shown in FIG. 2A, the locking member 504 may comprise a cylindrical body 506 configured to directly couple to the distal end 302b of the tubular body 302 and defining a passage 508 therethrough. The passage 508 may include at least one suture locking element configured to allow at least one repair suture to slide freely through the passage 508 in a first (e.g., distal) direction and to engage the at least one repair suture to prevent it from sliding in a second (e.g., proximal) direction. For example, the passage 508 may taper from a distal end 506b of the locking member body 506 to a proximal end 506a of the locking member body 506. A ball 510 may be displaceably disposed within the passage 508. The transfer member 400 may extend through the passage 508 of the locking member 504. The transfer member 400 may be configured to draw the repair suture through the passage in the first direction. As the repair suture is pulled distally through the passage 308, the ball 510 may be pushed into the wider portion of the passage 508, creating space for the repair suture to slide through. If the repair suture is moved proximally (i.e., tensioned), the ball 510 may be pushed into the narrower portion of the passage 508, wedging the repair suture between the ball 510 and an interior surface of the passage 508, effectively locking the repair suture within the passage 508. As shown in FIG. 2B, a pointed tip 512 on the locking member 504 may allow for a "self-punching" version of the locking member 504 that can penetrate tissue without the need for a pre-drilled hole.

Embodiments of the locking member body 506 and the ball 510 could be made of a lightweight, biocompatible material, such as titanium, or a dense polymer, such as polyether ether ketone (PEEK). The disclosure also contemplates that the locking member body 506 and ball 510 could be made of a bioabsorbable material if intended for temporary fixation. A spring or flexure could be included in the locking member 504 to apply pressure to the ball 510 in the proximal direction to ensure quicker locking with less suture displacement. Variations in the diameter of the ball 510 and the angle of the passage 508 may affect locking performance and the number of sutures that can be locked within the passage 508.

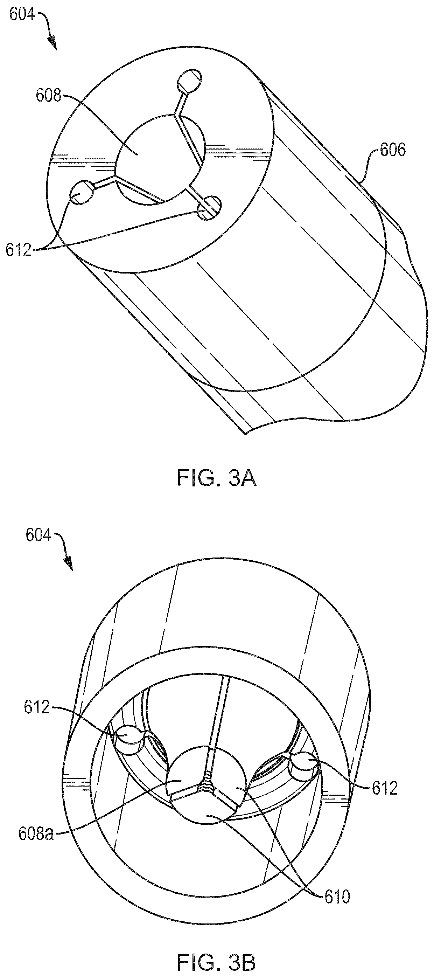

FIGS. 3A and 3B illustrate another alternative locking member 604 for use with the soft suture anchor assembly 100, according to an embodiment of the disclosure. FIG. 3A shows the locking member 604 as viewed from the distal end, while FIG. 3B shows the locking member 604 as viewed from the proximal end. As shown in FIG. 3A, the locking member 604 may comprise a cylindrical body 606 configured to directly couple to the distal end 302b of the tubular body 302 and defining a passage 608 therethrough. The passage 608 may include at least one suture locking element configured to allow at least one repair suture to slide freely through the passage 608 in a first (e.g., distal) direction and to engage the at least one repair suture to prevent it from sliding in a second (e.g., proximal) direction. The locking member body 606 may further include a plurality (e.g., three) slots 612 in communication with the passage 608 for housing the repair suture or sutures once final tension on the sutures has been placed. As shown in FIG. 3B, a proximal end 608a of the passage 608 may comprise a plurality (e.g., three) flexures 610 configured to allow passage of the repair suture distally through the passage 608, but to grip or trap the repair suture when pulled (i.e., tensioned) proximally, similar to the action of a CAM cleat, as further described below.

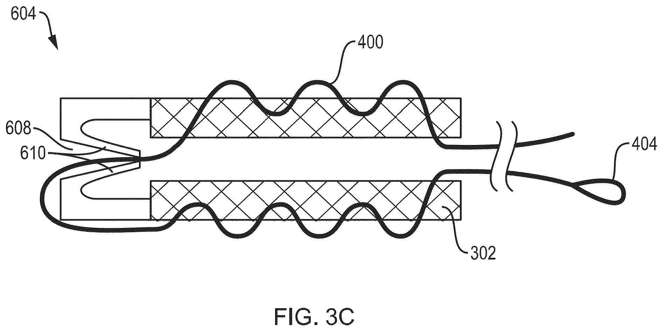

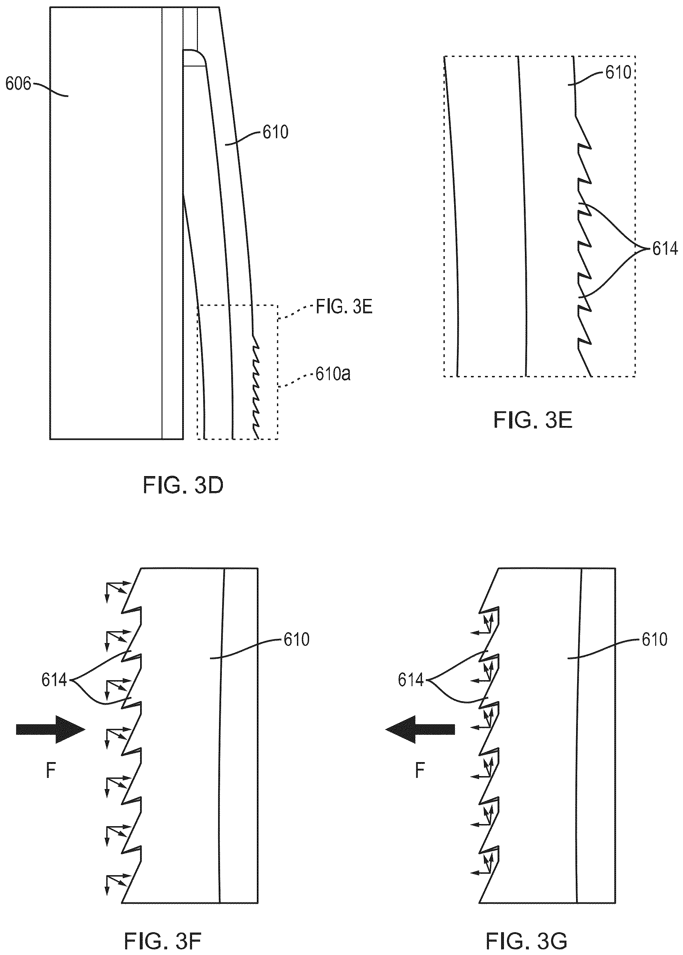

FIGS. 3C-3G illustrate the action of the flexures 610 of the locking member 604, according to an embodiment of the disclosure. As shown in FIGS. 3C, the flexures 610 may be flexibly coupled to the locking member body 606 and extend into an interior surface of the passage 608. As shown in FIGS. 3D and 3E, a proximal end 610a of the flexures 610 may include a plurality of gripping features 614, such as barbs, positioned to face each other about the passage 608. As shown in FIG. 3F, when the repair suture is pulled distally through the passage 608, a shape of the gripping features 614 may cause a net force F exerted by the pulling of the repair suture to move the flexures 610 away from the passage 608, reducing drag on the repair suture and allowing it to move freely through the passage 608. As shown in FIG. 3G, when the repair suture is pulled proximally through the passage 608 (i.e., tensioned), a shape of the gripping features 614 may cause the net force F to move the flexures 610 toward the passage 608, increasing drag on the repair suture and preventing it from moving freely through the passage 608. The disclosure also contemplates the use of rotatable or pivoting CAM elements, rather than the flexures 610.

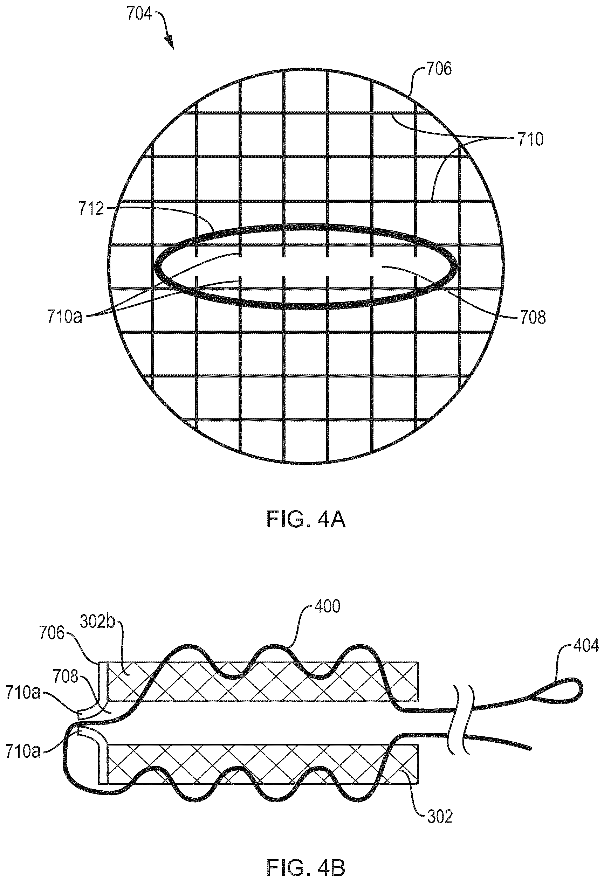

FIGS. 4A and 4B illustrate another alternative locking member 704 for use with the soft suture anchor assembly 100, according to an embodiment of the disclosure. As shown in FIGS. 4A and 4B, the locking member 704 may comprise a circular or elliptical body 706 in the form of a flat screen made up of a plurality of metal (e.g. stainless steel) wires 710 in a crosshatch or other suitable pattern. The locking member body 706 may be configured to directly couple to the distal end 302b of the tubular body 302 and may have a slit or elongated opening 712 in a mid-section of the locking member body 706 extending perpendicularly to one of the courses of wires 710. The opening 712 may define a passage 708 through the locking member body 706. The passage 708 may include at least one suture locking element configured to allow at least one repair suture to slide freely through the passage 708 in a first (e.g., distal) direction and to engage the at least one repair suture to prevent it from sliding in a second (e.g., proximal) direction. For example, the ends 710a of the wires 710 adjacent the passage 708 may be bent in a single direction. When the repair suture is pulled in a proximal direction (i.e., tensioned) friction on the ends 710a of the wires 710 may cause the wires 710 to grip the braided fibers of the repair suture, securing it into place. When the repair suture is pulled in the distal direction, the friction on the ends 710a of the wires allow the ends 710a of the wires 710 to release the braided fibers of the repair suture, allowing the repair suture to move more freely through the passage 708.

The disclosure contemplates the locking member body 706 could comprise a perforated disc rather than a wire screen. Furthermore, a pierced hole could be used rather than an elongated opening. Rather than a flat screen, a screen tube could be used. The shape of the screen could be any polygon, and multiple screens could be used. Two or more intersecting openings 712 could be used in place of the single opening 712. The screen could be made of a material other than metal, such as a biocompatible plastic.

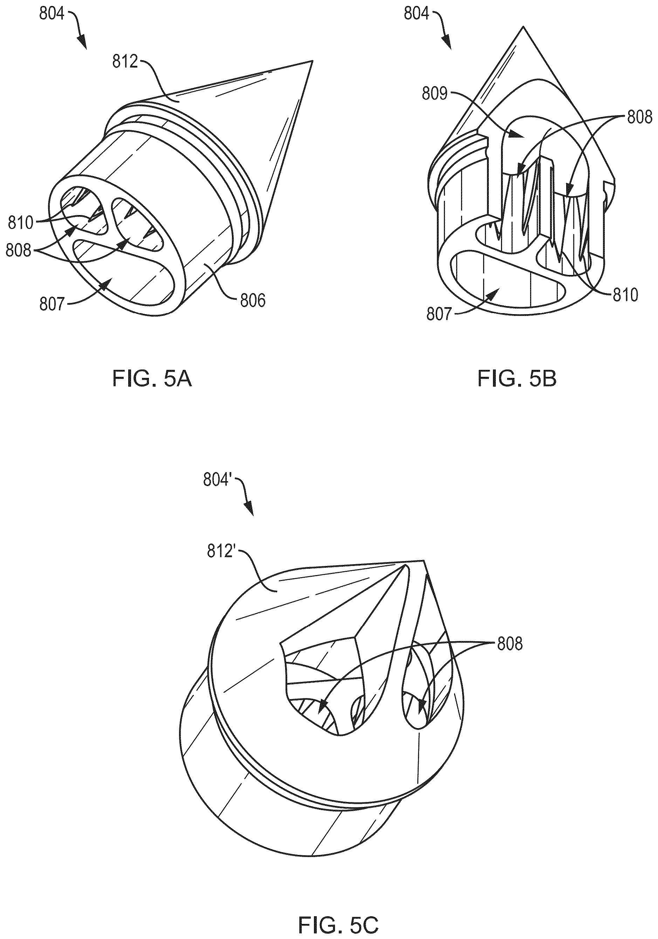

FIGS. 5A and 5B illustrate another alternative locking member 804 for use with the soft suture anchor assembly 100, according to an embodiment of the disclosure. As shown in FIGS. 5A and 5B, the locking member 804 may comprise a cylindrical or other suitably shaped body 806 configured to directly couple to the distal end 302b of the tubular body 302 of the soft anchor 300. The locking member body 806 may include a pointed tip 812 configured to penetrate tissue without the need for a pre-drilled hole. However, the disclosure also contemplates that the tip 812 of the locking member body 806 could be rounded or blunt. The locking member body 806 may define at least one through passage 807 and at least two suture capture passages 808 through the locking member body 806. The through passage 807 may be configured to allow at least one repair suture to slide freely through the through passage 807 and into a pass-through galley 809 in communication with both the through passage 807 and the suture capture passages 808. The pass-through galley 809 may then allow the least one repair suture to pass into one of the suture capture passages 808. The suture capture passages 808 may include at least one suture locking element configured to allow each separate repair suture to slide freely through the passage 808 in a first (e.g., proximal) direction and to resist the repair suture from sliding in a second (e.g., distal) and thus from being pulled back through the through passage 807. For example, an inner surface of the suture capture passages 808 may comprise at least one proximally extending spike or tooth 810. In embodiments, the at least one tooth 810 may be four teeth 810 as shown. However, the disclosure contemplates more or fewer than four teeth 810. The teeth 810 may be arranged about the inner surface of the suture capture passage 808 and extend toward a center of the passage 808. When the repair suture is pulled through passage 808 in the distal direction (e.g., tensioned), the teeth 810 may grip the braided structure of the repair suture firmly, securing it into place. When the repair suture is pulled through the passage 808 in the proximal direction, the teeth 810 may release the braided structure of the repair suture, enabling it to move more freely.

FIG. 5C illustrates an alternative configuration of the locking member 804', according to embodiments of the disclosure. As shown in FIG. 5C, the suture capture passages 808' may be formed entirely through the tip 812', facilitating molding of the locking member 804'. The disclosure contemplates that the suture capture passages 808' could be sealed with plugs (not shown) if needed.

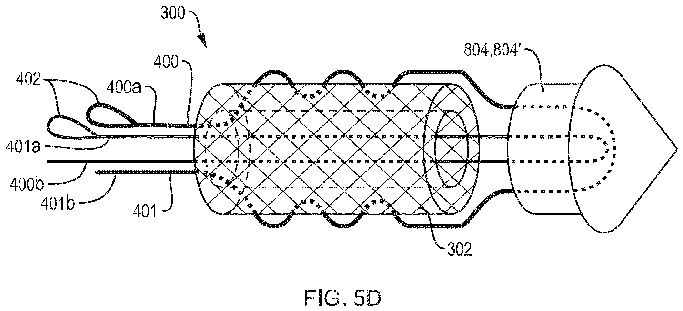

FIG. 5D is a schematic illustration of the soft anchor 300, the locking member 804, 804', and two transfers members 400, 401 according to an embodiment of the disclosure. Although two transfer members 400, 401 are shown in FIG. 5D, the disclosure contemplates more or fewer than two transfer members 400, 401 corresponding to the number of suture capture passages 808, 808'. As shown in FIG. 5D, each transfer member 400, 401 may be coupled to the tubular body 302 such that the transfer members 400, 401 extend along the tubular body 302 and through the locking member 804, 804'. For example, the transfer members 400, 401 may extend along a same first side of the tubular body 302 in a first direction and extend through the locking member 804, 804'. The transfer members 400, 401 may then extend along a second side of the tubular body 302 in a second direction opposite the first direction. First ends 400a, 401a of the transfer members 400, 401 may comprise a loop 402 or other coupling device for coupling to separate repair sutures. A user may first insert the locking member 804, 804' into tissue and then deploy the tubular body 302 to its expanded state to lock the soft anchor 300 into the surrounding tissue. The user may then couple the repair sutures to the loops 402 and then pull second ends 400b, 401b of the transfer members 400, 401 such that the repair sutures are pulled through the locking member 804, 804'. The repair sutures may then be used in the rest of the tissue repair.

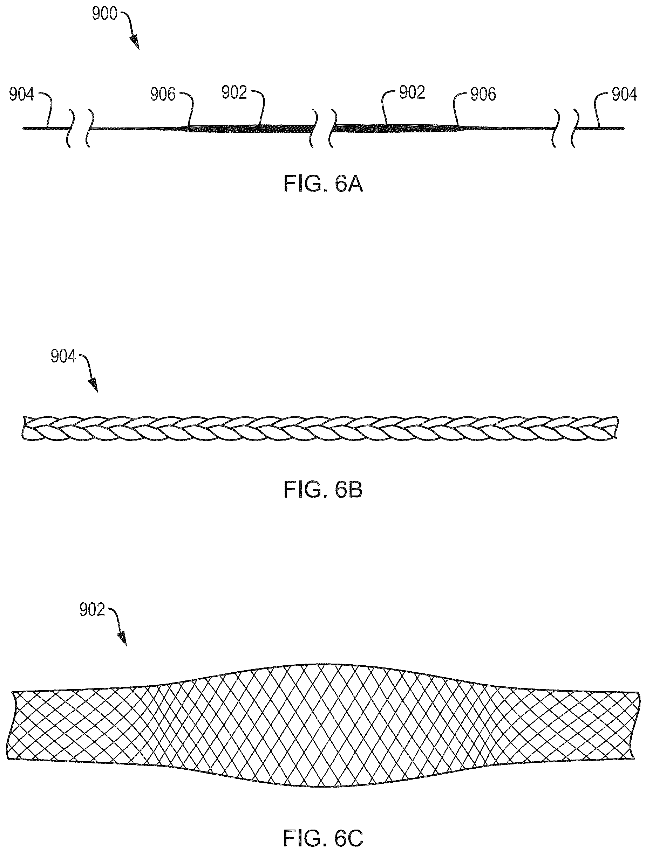

FIG. 6A is a schematic illustration of a repair suture 900 for use with the soft suture anchor assembly 100, according to an embodiment of the disclosure. The repair suture 900 may be configured to be more easily capturable by suture locking elements, such as the tabs 310 or teeth 810. For example, as shown in FIG. 6A, the repair suture 900 may comprise a middle region 902 and two end regions 904. A transition region 906 may extend between the middle region 902 and the two end regions 904. The end regions 904 may be intended to be folder over and pulled through a locking member (e.g., locking members 304, 604, 704, 804) using the transfer member 400. In embodiments, the two end regions 904 may comprise fewer carriers than the middle region 902. A "carrier" may be understood to refer to an individual strand or filament (which may itself be monofilament or multifilament) that is interlaced with other carriers in specific patterns to create the braided suture structure. For example, the end regions 904 may comprise nine carriers, while the middle region 902 may comprise 15 carriers. However, the disclosure contemplates more or fewer carriers for both the end regions 904 and the middle region 902. Moreover, the carriers of the end regions 904 may be braided at higher picks per inch (PPI) than the carriers of the middle region 902. PPI may be understood to measure the number of times the carriers cross over each other within one linear inch of the finished suture. For example, the carriers of the end regions 904 may be braided at a PPI of 63, while the carriers of the middle region 902 may be braided at a PPI of 32. However, the disclosure contemplates a higher or lower PPI for both the end regions 904 and the middle region 902.

FIGS. 6B and 6C are detailed illustrations of the end regions (FIG. 6B) and the middle region (FIG. 6C) of the repair suture 900, according to an embodiment of the disclosure. As shown in FIG. 6A, the fewer number of carriers braided at a higher PPI of the end regions 904 relative to the middle region 902 may facilitate manipulation of the end regions 904 with suture passers and other suture handling instruments. For example, the smaller diameter of the end regions 904 relative to the middle region 902 may allow the end regions 904 to occupy less space than standard sutures, while the higher PPI may result in a tighter braided structure, making the end regions 904 easier to handle than standard sutures using traditional suture manipulation instruments. In contrast, as shown in FIG. 6B, the looser configuration of the middle region 902 relative to the end regions 904 may enhance the ability of the middle region 902 to be securely captured by the suture locking elements (e.g., tabs 310, flexures 610, wires 710 or teeth 810). For example, when tension is applied to the repair suture 900, friction between the middle region 902 and the tabs 310 or teeth 810 may promote secure entrapment, ensuring that the repair suture 900 remains firmly anchored in place.

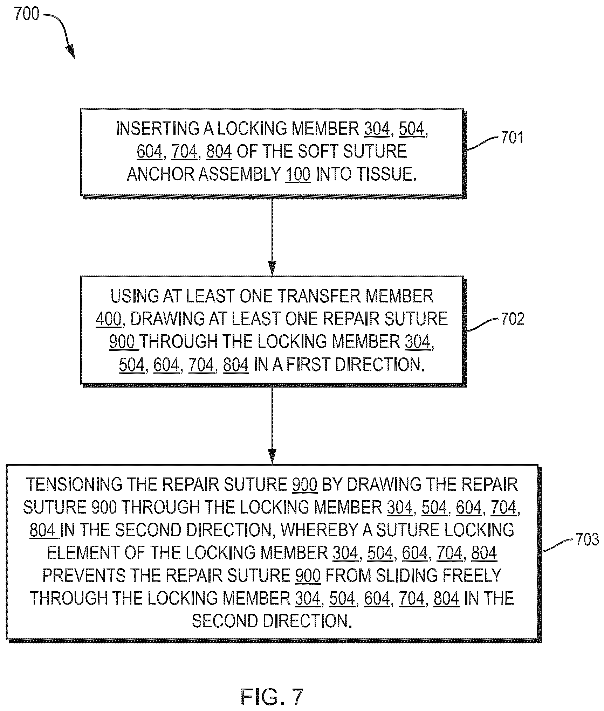

FIG. 7 is a flowchart of a method of tissue repair using the soft suture anchor assembly 100, according to an embodiment of the disclosure. As shown in FIG. 7, Step 701 comprises inserting a locking member 304, 504, 604, 704, 804 of the soft suture anchor assembly 100 into tissue. Step 702 comprises, using at least one transfer member 400, drawing at least one repair suture 900 through the locking member 304, 504, 604, 704, 804 in a first direction. Step 703 comprises tensioning the repair suture 900 by drawing the at least one repair suture 900 through the locking member 304, 504, 604, 704, 804 in a second direction, whereby a suture locking element of the locking member 304, 504, 604, 704, 804 prevents the at least one repair suture 900 from sliding freely through the locking member 304, 504, 604, 704, 804 the second direction.

While the disclosure particularly shows and describes preferred examples, those skilled in the art will understand that various changes in form and details may exist without departing from the spirit and scope of the present application as defined by the appended claims. The scope of this present application intends to cover such variations. As such, the foregoing description of examples of the present application does not intend to limit the full scope conveyed by the appended claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A soft suture anchor assembly for tissue repair, the soft suture anchor assembly comprising:
a soft anchor defining a tubular body;
a locking member comprising a locking member body coupled directly to and extending distally from a distal end of the tubular body, the locking member body defining at least one passage therethrough; and
at least one transfer member extending along the tubular body and through the at least one passage of the locking member, the at least one transfer member configured to draw at least one repair suture through the at least one passage in a first direction;
wherein the at least one passage is configured to allow the at least one repair suture to slide freely in the first direction through the at least one passage and to prevent the at least one repair suture from sliding freely in a second direction opposite the first direction through the at least one passage.

2. The soft suture anchor assembly of claim 1, the tubular body is configured to deploy from an elongated to an expanded state within tissue.

3. The soft suture anchor assembly of claim 2, wherein the at least one transfer member is configured to draw the at least one repair suture through the at least one passage in the first direction after deploying the tubular body to the expanded state.

4. The soft suture anchor assembly of any preceding claim, wherein the at least one transfer member is configured to draw the at least one repair suture through the at least one passage in the first direction after insertion of the locking member into tissue.

5. The soft suture anchor assembly of any preceding claim, wherein the at least one transfer member extends along and is interwoven through the tubular body and is configured to draw the at least one repair suture along and between braids of braided walls of the tubular body.

6. The soft suture anchor assembly of any preceding claim, wherein an end of the at least one transfer member is configured to couple to the at least one repair suture by a loop.

7. The soft suture anchor assembly of any preceding claim, wherein the repair suture originates from another knotless anchor.

8. The soft suture anchor assembly of any preceding claim, wherein the at least one passage comprises at least one suture locking element configured to engage the at least one repair suture to prevent the at least one repair suture from sliding freely in the second direction.

9. The soft suture anchor assembly of claim 8, wherein the at least one suture locking element is at least one tab, flexure, wire, or tooth on an inner surface of the at least one passage.

10. The soft suture anchor assembly of claim 8 or claim 9, wherein the at least one suture locking element is a ball configured to wedge the repair suture between the ball and an inner surface of the passage.

11. The soft suture anchor assembly of any preceding claim, wherein the at least one passage is at least one through passage in communication with at least one suture capture passage.

12. The soft suture anchor assembly of any preceding claim, wherein the locking member comprises a pointed tip.

13. The soft suture anchor assembly of any preceding claim, wherein the repair suture comprises a middle region extending between two end regions.

14. The soft suture anchor assembly of any preceding claim, wherein a number of carriers of two end regions is selected to be fewer than a number of carriers of a middle region, and wherein the carriers of the two end regions are braided at higher picks per inch (PPI) than the carriers of the middle region.

15. A method of tissue repair comprising:
inserting a locking member of a soft suture anchor assembly into tissue, the soft suture anchor assembly comprising:
a soft anchor defining a tubular body, the locking member comprising a locking member body coupled directly to and extending distally from a distal end of the tubular body, the locking member body defining at least one passage therethrough, the at least one passage configured to allow at least one repair suture to slide freely in a first direction through the at least one passage and to prevent the at least one repair suture from sliding freely in a second direction opposite the first direction through the at least one passage; and
at least one transfer member extending along the tubular body and through the at least one passage of the locking member;
using the transfer member, drawing at least one repair suture through the at least one passage of the locking member in the first direction; and
tensioning the repair suture by drawing the at least one repair suture through the at least one passage in the second direction, whereby a suture locking element of the locking member prevents the at least one repair suture from sliding freely through the passage in the second direction.
